Europäisches Patentamt

European Patent Office

Office européen des brevets

(11) Veröffentlichungsnummer: **0 297 157 B1**

(12) **EUROPÄISCHE PATENTSCHRIFT**

(45) Veröffentlichungstag der Patentschrift: **13.11.91**

(51) Int. Cl.⁵: **A61F 5/04**, A61F 5/01

(21) Anmeldenummer: **87109458.7**

(22) Anmeldetag: **01.07.87**

(54) Innenschuh zur Ruhigstellung des oberen und/oder des unteren Sprunggelenkes eines Fusses.

(43) Veröffentlichungstag der Anmeldung:
**04.01.89 Patentblatt 89/01**

(45) Bekanntmachung des Hinweises auf die Patenterteilung:
**13.11.91 Patentblatt 91/46**

(84) Benannte Vertragsstaaten:
**AT CH DE FR LI NL**

(56) Entgegenhaltungen:
**EP-A- 0 160 780**
**DE-A- 3 228 753**
**DE-U- 8 708 153**

(73) Patentinhaber: **Wetz, Hans-Henning, Dr. med.**
**Im Schwiendahl 46**
**W-5880 Lüdenscheid(DE)**

(72) Erfinder: **Wetz, Hans-Henning, Dr. med.**
**Im Schwiendahl 46**
**W-5880 Lüdenscheid(DE)**

(74) Vertreter: **Stark, Walter, Dr.-Ing.**
**Moerser Strasse 140**
**W-4150 Krefeld(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft einen Innenschuh zur Ruhigstellung des oberen und/oder des unteren Sprunggelenks eines Fußes, bestehend aus einer Kunststoffhülse aus einem hitzeverformbaren Kunststoff, die eine seitlich gelegene, sich über die gesamte Länge der Kunststoffhülse erstreckende Einstiegöffnung aufweist, wobei an die Kunststoffhülse eine steife Fersenkappe, die in eine das Fußlängsgewölbe stützende Bodenplatte übergeht, und außerdem eine Vorfußspannpelotte angeformt ist, die auf einer Seite in die Bodenplatte übergeht, während auf der gegenüberliegenden Seite sich die Einstiegöffnung durchgehend zwischen Vorfußspannpelotte und Bodenplatte erstreckt. - Eine Ruhigstellung des Sprunggelenkes ist erforderlich bei schwerem Verschleiß (Arthrose) oder nach Verletzungen und zur Umgehung bzw. Hinauszögerung einer Versteifungsoperation (Arthrodese). Die Ruhigstellung ist auch angezeigt bei Bandkapselverletzungen und zur Nachbehandlung von Bandkapselplastiken am oberen Sprunggelenk.

Bei einem bekannten Innenschuh der eingangs beschriebenen Gattung (DE-A-32 28 753) ist die eine Hälfte praktisch offen, so daß eine durchgehende Einstieg-Öffnung gebildet ist. Die Anwendung dieses bekannten Innenschuhs ist aber zumindest dann problematisch, wenn im Bereich des Sprunggelenks entzündliche oder traumatische Schwellungen vorliegen, weil der Innenschuh, jedenfalls auf der geschlossenen Seite im Bereich des Sprunggelenks,ebenfalls geschlossen ist, und ein Gurt sich gerade in diesem Bereich erstreckt.

Aufgabe der Erfindung ist es deshalb, einen gattungsgemäßen Innenschuh so zu verbessern, daß er auch bei entzündlichen oder traumatischen Schwellungen des Sprunggelenks eingesetzt werden kann.

Diese Aufgabe wird dadurch gelöst, daß die Einstiegöffnung im Bereich der Knöchelgabel verbreitert ist, daß auf der gegenüberliegenden Seite ein Knöchelfenster angeordnet ist und daß oberhalb der Verbreiterung der Einstiegöffnung eine versteifte Lasche angeordnet ist, die innenseitig an der Kunststoffhülse im Bereich des einen Öffnungsrandes befestigt ist und sich mit ihrem freien Ende über die Einstiegöffnung erstreckt.

Bei diesem Innenschuh bleiben die Bereiche des Sprunggelenks von Druckbeanspruchungen beim Tragen des Innenschuhs frei. Da im Bereich des Knöchelfensters auch keine Halteeinrichtungen oder Spanneinrichtungen angeordnet werden, andererseits aber für einen hinreichenden Halt und Sitz des Innenschuhs am Fuß bzw. Bein des Patienten gesorgt werden muß, ist oberhalb der Verbreiterung die versteifte Lasche vorgesehen, die es ermöglicht, mit geeigneten Verschlußeinrichtungen den Innen schuh oberhalb des Sprunggelenkes so festzulegen, daß ohne Beeinträchtigung des Tragekomforts, also Einklemmen von Hautpartien oder dergleichen, ein hinreichend fester Sitz erzielt wird. Im übrigen ermöglicht die seitlich gelegene Einstiegöffnung ein problemloses Anlegen des Innenschuhs. Die steife Fersenkappe ergibt in Verbindung mit der seitlichen Einstiegöffnung eine druckstellenfreie Abstützung des rückwärtigen Fußes bzw. der Ferse.

Oberhalb des Knöchelfensters kann ein sich in Hülsenlängsrichtung erstreckendes Fenster angeordnet sein, welches insbesondere eine ovale proximale mediale Öffnung bildet, die den lateralen Einstieg in den Innenschuh erleichtert und gleichzeitig auch als Weichteilfenster der medialen Abstützung dient.

Um einen festen Sitz der Kunststoffhülse am Fuß zu gewährleisten, sind vorzugsweise die Ränder der Einstiegöffnung über greifende Flächenreißverschlüsse vorgesehen. Mit Hilfe der Flächenreißverschlüsse können Abschnitte der Kunststoffhülse um die Weichteile gespannt werden.

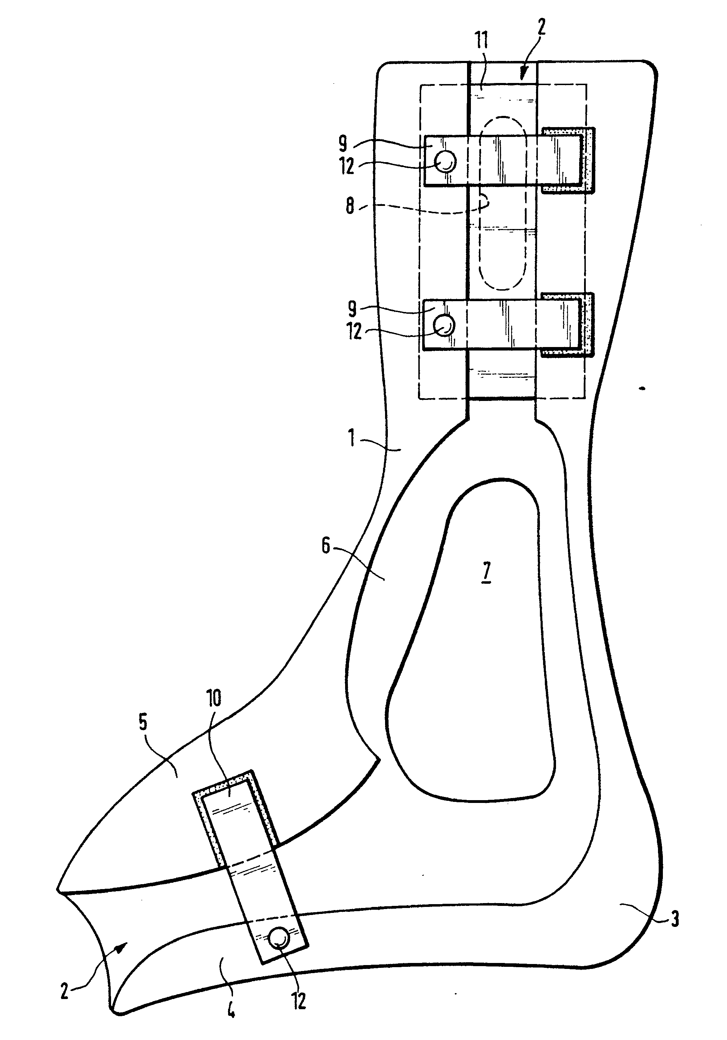

Im folgenden wird ein in der Zeichnung dargestelltes Ausführungsbeispiel der Erfindung erläutert; die einzige Figur zeigt die Ansicht eines Innenschuhs, von der Fußinnenseite her gesehen.

Der dargestellte Innenschuh dient zur Ruhigstellung des oberen und teilweise auch des unteren Sprunggelenks am Fuß eines Menschen bei schwerem Verschleiß, nach Verletzungen, zur Umgehung bzw. Hinauszögerung einer Versteifungsoperation und dergleichen. Der Innenschuh besteht aus einer Kunststoffhülse 1 aus hitzeverformbaren Kunststoff. Die Kunststoffhülse 1 ist dem zu behandelnden Fuß anatomisch angepaßt. Die Kunststoffhülse 1 weist fußinnenseitig eine Einstiegöffnung 2 auf, die sich über die gesamte Länge der Kunststoffhülse 1 erstreckt.

An die Kunststoffhülse 1 ist eine steife Fersenkappe 3 angeformt, die in eine das Fußlängsgewölbe stützende Bodenplatte 4 übergeht. An die Bodenplatte 4 schließt wiederum eine Vorfußspannpelotte 5 an, die sich über die Oberseite des Fußes erstreckt, wobei jedoch fußinnenseitig die Einstiegöffnung 2 zwischen Bodenplatte 4 und Vorfußspannpelotte 5 verbleibt. Die Bodenplatte 4 und die Vorfußspannpelotte 5 erstrecken sich bei angelegtem Innenschuh etwa bis zur Reihe der Mittelfußköpfchen. Die Schafthöhe des Innenschuhs insgesamt beträgt ca. 25 cm bei einer Schuhgröße von 40 bis 41.

Im Bereich der Knöchelgabel weist die Einstiegöffnung eine Verbreiterung 6 auf, die sich etwa über eine Höhe von 10 cm erstreckt. Auf der gegenüberliegenden Seite der Kunststoffhülse 1 ist ein Knöchelfenster 7 vorgesehen, dessen Abmessungen nur geringfügig kleiner sind als die Abmessungen der Verbreiterung 6. Dadurch liegt die Knöchelgabel frei und gestattet die Anwendung des Innenschuhs auch bei entzündlichen und traumatischen Schwellungen des Sprunggelenks.

Oberhalb des Knöchelfensters 7 befindet sich im Schaftbereich und der Einstiegöffnung 2 gegenüberliegend ein sich in Hülsenlängsrichtung erstreckendes ovales Fenster 8, welches etwa 10 cm lang und 3 cm breit ist. Dieses ovale proximale mediale Fenster 8 erleichtert den lateralen Einstieg in den Innenschuh und dient auch als Weichteilfenster der medalen Abstützung.

Es versteht sich, daß das Material der Kunststoffhülse 1 jedenfalls bei Umgebungstemperatur eine elastischnachgiebige Verformung derart zuläßt, daß der Innenschuh durch Aufspreizen der Einstiegöffnung 2 angelegt werden kann. Zur Verspannung des Innenschuhs am Fuß dienen Flächenreißverschlüsse 9, 10. Bei der dargestellten Ausführung sind jeweils zwei Flächenreißverschlüsse 9 im Schaftbereich vorgesehen, die den Innenschuh mit dem unteren Drittel des Unterschenkels und mit dem oberen Sprunggelenk verspannen sowie ein Flächenreißverschluß 10, der die Vorfußspannpelotte 5 mit der Bodenplatte 4 verspannt.

Im Bereich der beiden Flächenreißverschlüsse 9, d. h. oberhalb der Verbreiterung 6 der Einstiegöffnung 2 ist eine versteifte Lasche 11 vorgesehen, die innenseitig an der Kunststoffhülse 1 im Bereich des einen Öffnungsrandes befestigt ist und sich mit ihrem freien Ende über die Einstiegöffnung 2 erstreckt. Diese versteifte Lasche 11 schützt entsprechende Weichteile vor Einklemmen zwischen den Öffnungsrändern der Einstiegöffnung 2. Die Lasche 11 ist bei der dargestellten Ausführung mit Nieten 12 befestigt, von denen jeweils auch der eine Teil der Flächenreißverschlüsse 9 gehalten ist.

## Patentansprüche

1. Innenschuh zur Ruhigstellung des oberen und/oder des unteren Sprunggelenks eines Fußes, bestehend aus einer Kunststoffhülse (1) aus einem hitzeverformbaren Kunststoff, die eine seitlich gelegene, sich über die gesamte Länge der Kunststoffhülse (1) erstreckende Einstiegöffnung (2) aufweist, wobei an die Kunststoffhülse (1) eine steife Fersenkappe (3), die in eine das Fußlängsgewölbe stützende Bodenplatte (4) übergeht und außerdem eine Vorfußspannpelotte (5) angeformt ist, die auf einer Seite in die Bodenplatte (4) übergeht, während auf der gegenüberliegenden Seite sich die Einstiegöffnung (2) durchgehend zwischen Vorfußspannpelotte (5) und Bodenplatte (4) erstreckt, dadurch gekennzeichnet, daß die Einstiegöffnung (2) im Bereich der Knöchelgabel verbreitert ist, daß auf der gegenüberliegenden Seite ein Knöchelfenster (7) angeordnet ist und daß oberhalb der Verbreiterung (6) der Einstiegöffnung (2) eine versteifte Lasche (11) angeordnet ist, die innenseitig an der Kunststoffhülse (1) im Bereich des einen Öffnungsrandes befestigt ist und sich mit ihrem freien Ende über die Einstiegöffnung (2) erstreckt.

2. Innenschuh nach Anspruch 1, dadurch gekennzeichnet, daß oberhalb des Knöchelfensters (7) ein sich in Hülsenlängsrichtung erstreckendes Fenster (8) angeordnet ist.

3. Innenschuh nach einem der Ansprüche 1 oder 2, gekennzeichnet durch die Ränder der Einstiegöffnung (2) übergreifende Flächenreißverschlüsse (9, 10) im Bereich oberhalb der Verbreiterung (6) der Einstiegöffnung (2) sowie zwischen Vorfußspannpelotte (5) und Bodenplatte (4).

## Claims

1. An inner shoe for immobilising the upper and/or lower ankle joint of a foot, comprising a plastics sleeve (12) made of a heat-deformable plastics material, having a laterally-situated access opening (2) extending over the entire length of the plastics sleeve (1), there being moulded on to the plastics sleeve (1) a rigid heel cap (3), which merges into a baseplate (4) supporting the longitudinal arch of the foot, and also an instep tensioning pad (5) which merges on one side into the baseplate (4) while, on the opposite side, the access opening (2) extends continuously between the instep tensioning pad (5) and the baseplate (4), characterised in that the access opening (2) is widened in the region of the ankle fork, that on the opposite side there is disposed an ankle opening (7), and that there is disposed, above the widened portion (6) in the access opening (2), a reinforced lug (11) which is attached internally to the plastics sleeve (1) in the region of one edge of the opening, and extends with its free end across the access opening (2).

2. An inner shoe according to Claim 1, characterised in that there is disposed, above the ankle opening (7) an opening (8) extending longitudinally along the sleeve.

3. An inner shoe according to one of Claims 1 or 2, characterised by surface zip fasteners (9, 10) extending over the edges of the access opening (2) in the region above the widened portion (6) of the access opening (2) and also between the instep tensioning pad (5) and the baseplate (4).

## Revendications

1. Chausson intérieur, pour immobiliser l'articulation de la cheville, composé d'une gaine en matière synthétique (1) thermoformable, qui présente une ouverture de chaussement (2) latérale, qui s'étend sur toute la longueur de la gaine en matière synthétique (1), une coiffe de talon (3) rigide, qui se transforme en plaque de fond (4) servant d'appui à la voûte plantaire, et en outre une pelote de serrage de l'avant-pied (5), qui se transforme d'un côté en donnant la plaque de fond (4), étant formés d'un seul tenant sur la gaine en matière synthétique (1), tandis que, sur le côté opposé, l'ouverture de chaussement (2) s'étend de manière continue entre la pelote de serrage de l'avant-pied (5) et la plaque de fond (4), caractérisé en ce que l'ouverture de chaussement (2) est élargie dans la zone de la cheville, une fenêtre de cheville (7) étant disposée du côté opposé, une attache rigidifiée (11) étant disposée au-dessus de l'élargissement (6) de l'ouverture de chaussement (2) et fixée intérieurement sur la gaine en matière synthétique (1), dans la zone d'un bord d'ouverture et s'étendant avec son extrémité libre au-dessus de l'ouverture de chaussement (2).

2. Chausson intérieur selon la revendication 1, caractérisé en ce qu'au-dessus de la fenêtre de cheville (7) est disposé une fenêtre (8) qui s'étend en direction longitudinale de la gaine.

3. Chausson intérieur selon l'une quelconque des revendications 1 ou 2, caractérisé par des obturations à surfaces à déchirer (9,10), entourant les bords de l'ouverture de chaussement (2), dans la zone située au-dessus de l'élargissement (6) de l'ouverture de chaussement (2), ainsi qu'entre la pelote de serrage de l'avant pied (5) et la plaque de fond (4).